# EUROPÄISCHE PATENTSCHRIFT

(11) **EP 2 240 458 B1**
(45) Veröffentlichungstag und Bekanntmachung des Hinweises auf die Patenterteilung: **03.08.2011**
(21) Anmeldenummer: 09708163.2
(22) Anmeldetag: 05.02.2009
(51) Int. Cl.: C07D 295/125, C07D 401/04, C07D 401/06, C07D 403/06, C07D 407/14, A61K 31/18, A61P 25/04

(54) **ARYLSULFONAMIDE WIRKSAM ALS SCHMERZMITTEL**
ARYL SULFONAMIDES AS EFFECTIVE ANALGESICS
ARYLSULFONAMIDES À EFFICACITÉ ANTALGIQUE

(30) Priorität: 06.02.2008 EP 08101319
(43) Veröffentlichungstag der Anmeldung: 20.10.2010
(73) Patentinhaber: Boehringer Ingelheim International GmbH, 55216 Ingelheim am Rhein (DE)
(72) Erfinder: HAUEL, Norbert, 55216 Ingelheim am Rhein (DE); CECI, Angelo, 55216 Ingelheim am Rhein (DE); DOODS, Henri, 55216 Ingelheim am Rhein (DE); KAUFFMANN-HEFNER, Iris, 55216 Ingelheim am Rhein (DE); KONETZKI, Ingo, 55216 Ingelheim am Rhein (DE); SCHULER-METZ, Annette, 55216 Ingelheim am Rhein (DE); WALTER, Rainer, 55216 Ingelheim am Rhein (DE)
(74) Vertreter: Hammann, Heinz
(86) Internationale Anmeldenummer: PCT/EP2009/000768
(87) Internationale Veröffentlichungsnummer: WO 2009/098051

(56) Entgegenhaltungen:
- WO-A-97/25315
- US-A1- 2006 084 699

## Beschreibung

Gegenstand der vorliegenden Erfindung sind Verbindungen der allgemeinen Formel **I** 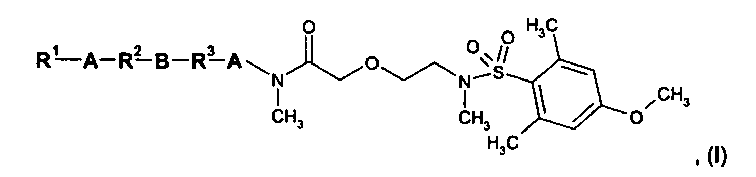 in der **A, B, R¹, R²** und **R³** wie nachstehend erwähnt definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

### DETAILLIERTE BESCHREIBUNG DER ERFINDUNG

In der obigen allgemeinen Formel I bedeuten in einer ersten Ausführungsform
- **A**: (a) eine Bindung oder
(b) eine C₁₋₃-Alkylengruppe,
- **B**: (a) eine Bindung,
(b) eine C₁₋₃-Alkylen- oder
(c) eine -C(O)-Gruppe,
- **R¹**: (a) H,
(b) C₁₋₃-Alkyl,
(c) C₃₋₆-Cycloalkyl,
(d) einen 5- oder 6-gliedrigen, gesättigten Aza-Heterocyclus oder
(e) einen 5- oder 6-gliedrigen, gesättigten Oxa-Heterocyclus,
- **R**²: (a) C₁₋₃-Alkylen,
(b) einen 4-bis 6-gliedrigen, gesättigten Aza-Heterocyclus, oder
(c) einen 6- bis 7-gliedrigen, gesättigten Diaza-Heterocyclus,
- **R³**: (a) C₂₋₄-Alkenylen,
(b) C₂₋₄-Alkinylen oder
(c) einen 5- bis 6-gliedrigen, gesättigten Aza-Heterocyclus, in dem zusätzlich eine Methylengruppe durch eine Carbonylgruppe substituiert sein kann,
deren Enantiomere, deren Diastereomere, deren Gemische und deren Salze, insbesondere deren physiologisch verträgliche Salze mit organischen oder anorganischen Säuren oder Basen.

Eine zweite Ausführungsform der vorliegenden Erfindung besteht in den Verbindungen der obigen allgemeinen Formel I, in denen
- **A**: eine Bindung oder eine -CH₂-Gruppe,
- **B**: eine Bindung, eine -CH₂- oder -C(O)-Gruppe,
- **R¹**: H, H₃C- oder eine Gruppe ausgewählt aus 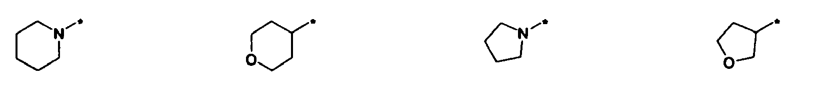 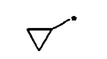
- **R²**: eine Gruppe ausgewählt aus 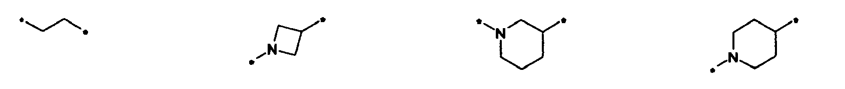 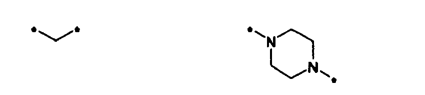 und
- **R³**: eine Gruppe ausgewählt aus 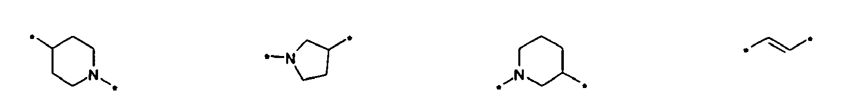 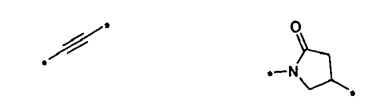
bedeutet,
deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel I seien beispielsweise folgende genannt:

| **Beispiel** | **Struktur** |
|---|---|
| (1) | 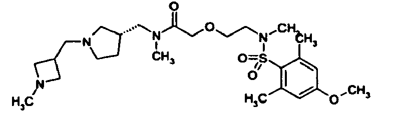 |
| (2) | 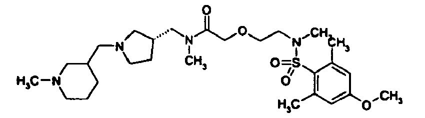 |
| (3) | 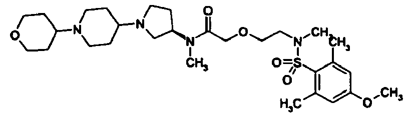 |
| (4) | 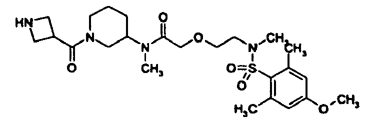 |
| (5) | 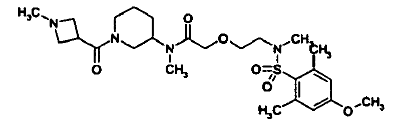 |
| (6) | 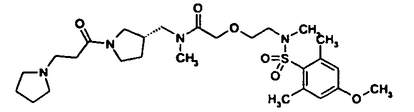 |
| (7) | 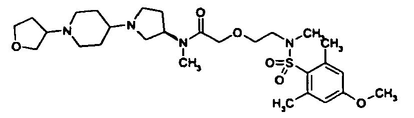 |
| (8) | 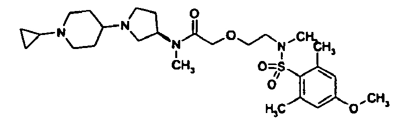 |
| (9) | 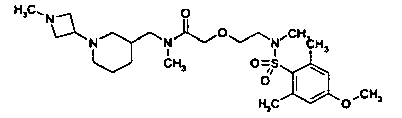 |
| (10) | 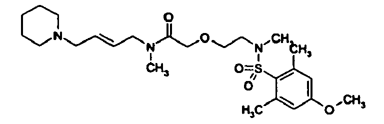 |
| (11) | 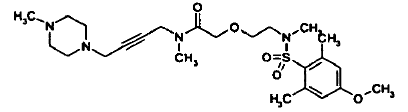 |
| (12) | 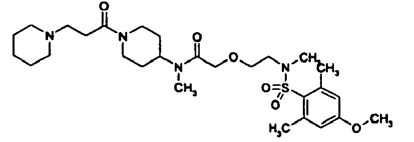 |
| (13) | 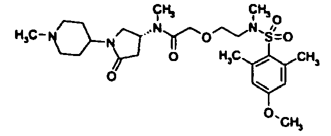 |

deren Enantiomere, deren Diastereomere, deren Gemische und deren Salze, insbesondere deren physiologisch verträgliche Salze mit organischen oder anorganischen Säuren oder Basen.

### VERWENDETE BEGRIFFE UND DEFINITIONEN

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden Alkylgruppen mit 1 bis 3 Kohlenstoffatomen verstanden. Beispielsweise werden hierfür genannt: Methyl, Ethyl, n-Propyl und iso-Propyl. Gegebenenfalls werden für die vorstehend genannten Gruppen auch die Abkürzungen Me, Et, n-Pr oder i-Pr verwendet. Sofern nicht anders beschrieben, umfaßt die Definition Propyl alle denkbaren isomeren Formen des Restes, beispielsweise Propyl, n-Propyl und iso-Propyl.
Weiterhin umfassen die voranstehend genannten Begriffe auch solche Reste, in denen jede Methylengruppe mit bis zu zwei und jede Methylgruppe mit bis zu drei Fluoratomen substituiert sein kann.

Unter dem Begriff "C₁₋₃-Alkylen" werden verzweigte und unverzweigte Alkylengruppen mit 1 oder 3 Kohlenstoffatomen verstanden. Beispielsweise werden hierfür genannt: Methylen, Ethylen, Ethan-1,1-diyl und Propylen. Sofern nicht anders beschrieben, umfaßt die Definition Propylen alle denkbaren isomeren Formen der gleichen Kohlenstoffanzahl, beispielsweise 1-Methylethylen.
Weiterhin umfassen die voranstehend genannten Begriffe auch solche Reste, in denen jede Methylengruppe mit bis zu zwei Fluoratomen substituiert sein kann.

Unter dem Begriff "C₂₋₄-Alkenylen" (auch soweit sie Bestandteil anderer Reste sind) werden verzweigte und unverzweigte Alkenylengruppen mit 2 bis 4 Kohlenstoffatomen verstanden. Beispielsweise werden hierfür genannt: Ethenylen, Propenylen, 1-Methylethenylen, Butenylen, 1-Methylpropenylen, 1,1-Dimethylethenylen oder 1,2-Dimethylethenylen. Sofern nicht anders beschrieben, umfassen die Definitionen Propenylen und Butenylen alle denkbaren isomeren Formen der jeweiligen Reste gleicher Kohlenstoffanzahl. So umfasst beispielsweise Propenyl auch 1-Methylethenylen und Butenylen umfasst 1-Methylpropenylen, 1,1-Dimethylethenylen, 1,2-Dimethylethenylen.

Unter dem Begriff "C₂₋₄-Alkinylen" (auch soweit sie Bestandteil anderer Reste sind) werden verzweigte und unverzweigte Alkinylengruppen mit 2 bis 4 Kohlenstoffatomen verstanden. Beispielsweise werden hierfür genannt: Ethinylen, Propinylen, 1-Methylethinylen, Butinylen, 1-Methylpropinylen, 1,1-Dimethylethinylen oder 1,2-Dimethylethinylen. Sofern nicht anders beschrieben, umfassen die Definitionen Propinylen und Butinylen alle denkbaren isomeren Formen der jeweiligen Reste gleicher Kohlenstoffanzahl. So umfasst beispielsweise Propinyl auch 1-Methylethinylen und Butinylen umfasst 1-Methylpropinylen, 1,1-Dimethylethinylen, 1,2-Dimethylethinylen.

Unter dem Begriff "C₃₋₆-Cycloalkyl" (auch soweit sie Bestandteil anderer Reste sind) werden cyclische Alkylgruppen mit 3 bis 6 Kohlenstoffatomen verstanden. Beispielsweise werden hierfür genannt: Cyclopropyl, Cyclobutyl, Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso-*Propyl, tert-Butyl, Hydroxy, Fluor, Chlor, Brom und Jod.

Unter dem Begriff "gesättigte Aza-Heterocyclen" werden vier-, fünf- oder sechsgliedrige heterocyclische Ringe verstanden, die ein Stickstoffatom enthalten. Dabei ist der Ring entweder über das Stickstoffatom oder über das Stickstoffatom und ein Kohlenstoffatom mit dem restlichen Molekül verknüpft. Als Beispiele werden genannt:

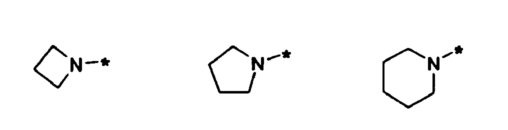

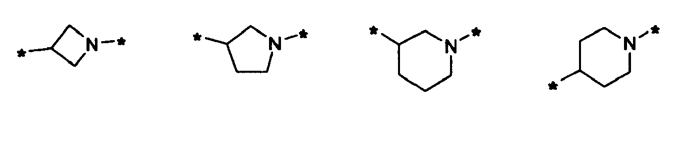

Unter dem Begriff "gesättigte Diaza-Heterocyclen" werden sechs- oder siebengliedrige heterocyclische Ringe verstanden, die zwei Stickstoffatome enthalten. Dabei ist der Ring über beide Stickstoffatome mit dem restlichen Molekül verknüpft. Als Beispiele werden genannt:

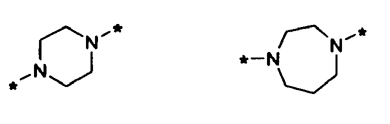

Unter dem Begriff "gesättigte Oxa-Heterocyclen" werden fünf- oder sechsgliedrige heterocyclische Ringe verstanden, die ein Sauerstoffatom enthalten. Dabei ist der Ring über ein Kohlenstoffatom mit dem restlichen Molekül verknüpft. Als Beispiele werden genannt:

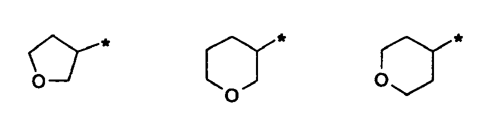

Verbindungen der allgemeinen Formel I können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oder *p*-Toluolsulfonsäure in Frage, als organische Säuren kommen beispielsweise Äpfelsäure, Bernsteinsäure, Essigsäure, Fumarsäure, Maleinsäure, Mandelsäure, Milchsäure, Weinsäure oder Zitronensäure in Betracht. Weiterhin können gegebenenfalls im Molekül vorhandene tertiäre Aminogruppen quarternisiert werden. Zur Umsetzung werden dabei Alkylhalogenide eingesetzt. Erfindungsgemäß bevorzugt wird für die Quartenisierung Methyliodid verwendet.

Weiterhin lassen sich die Verbindungen der allgemeinen Formel I, sofern sie geeignete Carbonsäurefunktionen enthalten, gewünschtenfalls in ihre Additionssalze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.

Die erfindungsgemäßen Verbindungen können als Racemate vorliegen, sofern sie nur ein Chiralitätselement besitzen, sie können aber auch als reine Enantiomere, d.h. in (*R*)- oder (*S*)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel I vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### HERSTELLVERFAHREN

Erfindungsgemäß erhält man die Verbindungen der allgemeinen Formel I nach an sich bekannten Verfahren, beispielsweise nach folgenden Verfahren:

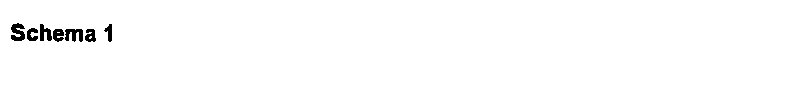

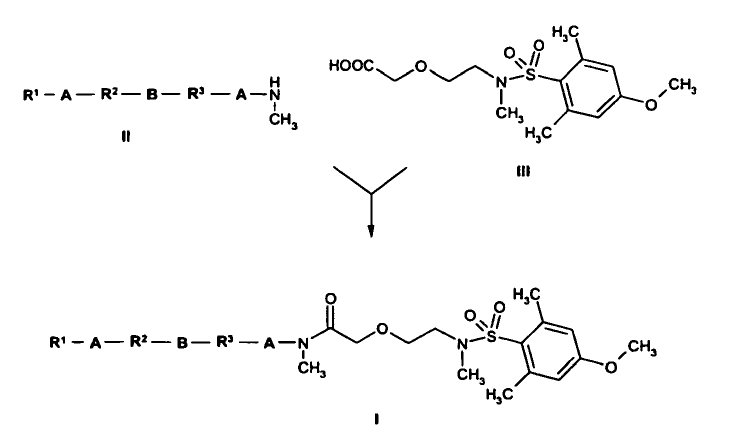

Die Amin-Bausteine der allgemeinen Formel **II,** in der alle Reste wie voranstehend erwähnt definiert sind, werden nach literaturbekannten Verfahren hergestellt.
Die in Schema 1 dargestellte Verknüpfung einer Carbonsäure der allgemeinen Formel **III,** in der alle Reste wie voranstehend erwähnt definiert sind, mit einem Amin der allgemeinen Formel II, in der alle Reste wie voranstehend erwähnt definiert sind, unter Bildung eines Carbonsäureamids der allgemeinen Formel **I,** in der alle Reste wie voranstehend erwähnt definiert sind, kann mit herkömmlichen Methoden zur Amidbildung durchgeführt werden.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z.B. Dicyclohexylcarbodiimid (DCC), Diisopropylcarbodiimid (DIC) oder Ethyl-(3-dimethylamino-propyl)-carbodiimid, *O*-(1*H*-Benzotriazol-1-yl)-*N,N-N',N'*-tetramethyluronium-hexafluorphosphat (HBTU) oder -tetrafluorborat (TBTU) oder 1*H*-Benzotriazol-1-yl-oxy-tris-(dimethylamino)-phosphonium-hexafluorphosphat (BOP) eingesetzt werden. Durch Zugabe von 1-Hydroxybenzotriazol (HOBt) oder von 3-Hydroxy-4-oxo-3,4-di-hydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösemitteln wie Dichlormethan, Tetrahydrofuran (THF), Acetonitril, Dimethylformamid (DMF), Dimethylacetamid (DMA), *N*-Methylpyrrolidon (NMP) oder Gemischen aus diesen und bei Temperaturen zwischen -30°C und +30°C, bevorzugt -20°C und +25°C, durchgeführt. Sofern erforderlich, wird als zusätzliche Hilfsbase Diisopropylethylamin (DIPEA) (Hünig-Base) bevorzugt.

### Methodenbeschreibung zur Bradvkinin BK1-Rezeptorbindung

CHO-Zellen, die den Ratten-BK1-Rezeptor stabil exprimieren, werden in "Dulbecco's modified Medium" kultiviert. Von konfluenten Kulturen wird das Medium entfernt, die Zellen werden mit PBS-Puffer gewaschen, abgeschabt und durch Zentrifugieren isoliert. Anschließend werden die Zellen in Suspension homogenisiert, das Homogenat zentrifugiert und resuspendiert. Nach Bestimmung des Proteingehalts wird die so erhaltene Membranpräparation bei -80°C eingefroren.
Nach dem Auftauen werden 200 µl des Homogenats (50 bis 250 µg Protein/Assay) für 120 Minuten bei Raumtemperatur mit 1 bis 5 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCount NXT (Perkin Elmer/Packard) gemessen. Als nichtspezifische Bindung wird die gebundene Radioaktivität in Gegenwart von 1.0 µM Lys-Des-Arg9-bradykinin definiert. Die Analyse der Konzentrations-Bindungskurve erfolgt mit Hilfe einer computergestützten nichtlinearen Kurvenanpassung. Aus den so erhaltenen Daten wird für die Testsubstanz der entsprechende Kᵢ-Wert ermittelt.
Um zu zeigen, dass die Verbindungen der allgemeinen Formel I mit unterschiedlichen Strukturelementen gute Bradikinin-B1-Rezeptor antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht um spezifische Verbindungen hervorzuheben:

| **Beispiel** | **Kᵢ [nM]** |
|---|---|
| (3) | 24.7 |
| (6) | 181 |
| (8) | 23.5 |

### INDIKATIONEN

Aufgrund ihrer pharmakologischen Eigenschaften eignen sich die neuen Verbindungen und deren physiologisch verträglichen Salze zur Behandlung von Krankheiten und Krankheitssymptomen, die wenigstens teilweise durch die Stimulierung von Bradykinin-B1-Rezeptoren hervorgerufen werden.
Aufgrund ihrer pharmakologischen Wirkung eignen sich die Substanzen zur Behandlung
(a) von akuten Schmerzen wie z.B. Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) von Eingeweideschmerzen wie z.B. chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, bei Angina pectoris, Schmerzen bei Kolon irritabile einschließlich der Crohnschen Krankheit und der ulcerativen Kolitis, bei nicht-ulzeröser Dyspepsie und bei Gastritis, Prostatitis, nicht-kardialen Thoraxschmerzen und Schmerzen bei myokardialer Ischämie und Herzinfarkt, Schmerzen bei Koliken, Nephritis, Uveitis;
(c) von neuropathischen Schmerzen wie z.B. schmerzhaften Polyneuropathien, Rückenschmerzen, Schmerzen bei diabetischer Neuropathie, Schmerzen nach einem Schlaganfall, AIDS-assoziierten neuropathischen Schmerzen, Herpes zoster induzierten Schmerzen, Schmerzen bei Lumbago, bei nicht-herpes-assoziierter Neuralgie, bei Postzoster Neuralgie, bei Nervenverletzungen, bei Schädel-Hirn-Trauma, Schmerzen bei Nervenschädigungen infolge von Toxinen oder Chemotherapie, bei Phantomschmerzen, Schmerzen bei multipler Sklerose, bei Nervenwurzelabriß und schmerzhaften traumatisch bedingten Einzelnervenschädigungen bei dem Carpal-Turnnel-Syndrom, bei der ulnaren Neuropathie, bei Radiculopathie, bei Hyperalgesie oder Allodynie assozierten Schmerzen;
(d) von entzündlichen / Schmerzrezeptor-vermittelten Schmerzen im Zusammenhang mit Erkrankungen wie Osteoarthritis, rheumatoider Arthritis, rheumatischem Fieber, Tendo-Synovitis, Sehnenentzündungen, Gicht, Vulvodynie, Schädigungen und Erkrankungen der Muskeln und Faszien (muskuläre Verletzungen, Fibromyalgie), atopischer Dermatitis, Psoriasis, Ekzemen, cerebralen Ödemen, Angioödemen, Crohnscher Krankheit, Pelvitis" juveniler Arthritis, Spondylitis, Gicht-Arthritis, Psoriasis-Arthritis, Fibromyalgie, Myositis, Migräne, Zahnerkrankungen, Influenza und anderen Virusinfektionen wie Erkältungen, bakteriellen Infektionen, Verspannungen, Prellungen, Verstauchungen, Frakturen oder systemischer Lupus erythematodes,
(e) von Tumorschmerzen im Zusammenhang mit Krebserkrankungen wie lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) von Kopfschmerz-Erkrankungen wie z.B. Kopfschmerzen unterschiedlicher Ursache, Cluster-Kopfschmerz, Migräne (mit oder ohne Aura) und Spannungskopfschmerz.

Weiterhin eigenen sich die Verbindungen zur Behandlung
(g) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nicht-allergisch bedingten asthmatischen Erkrankungen;
   chronisch obstruktiver Lungen-Erkrankung (COPD) einschließlich Lungenemphysem, akutem Atemnotsyndrom des Erwachsenen (ARDS), Bronchitis, Lungenentzündung, cystischer Fibrose, allergischer Rhinitis (saisonal und ganzjährig) und Sinusitis, nichtallergischer Sinusitis" vasomotorischer Rhinitis und Staublungen-Erkrankungen wie Aluminose, Anthrakose, Asbestose, Chalikose, Siderose, Silikose, Tabakose und Byssinose;
(h) von Entzündungserscheinungen bei Sonnenbrand und Verbrennungen, Ödemen nach Traumata durch Verbrennungen, Hirnödemen und Angioödemen, Darmerkrankungen einschließlich Morbus Crohn und Kolitis ulzerosa, Reizdarmsyndrom, Pankreatitis, Nephritis, Zystitis (interstitielle Zystitis), Uveitis; entzündlichen Erkrankungen der Haut (wie z.B. Angioderma, Psoriasis und Ekzeme), vaskulären Bindegewebserkrankungen, Lupus, Zerrungen und Frakturen;
(i) von Diabetes Mellitus und dessen Folgen (wie z.B. diabetische Vaskulopathie, diabetische Neuropathie, diabetische Retinopathie) und von diabetischen Symptomen bei Insulitis (z.B. Hyperglycämie, Diurese, Proteinurie und erhöhte renale Nitrit- und Kallikrein-Exkretion);
(j) von neurodegenerativen Erkrankungen wie der Parkinson'schen Erkrankung und der Alzheimer Erkrankung;
(k) von Sepsis und septischem Schock nach bakteriellen Infektionen oder nach Trauma;
(I) von Juckreiz verursachenden Syndromen und allergischen Hautreaktionen;
(m) von Osteoporose;
(n) von Epilepsie;
(o) von Verletzungen des Zentralnervensystems;
(p) von Wunden und Gewebeschädigungen;
(q) von Zahnfleischentzündungen;
(r) von benigner Prostata-Hyperplasie und hyperaktiver Blase;
(s) von Pruritus;
(t) von Vitiligo;
(u) von Störungen der Motilität von respiratorischen, genito-urinalen, gastro-intestinalen oder vaskulären Regionen,
(v) von post-operativem Fieber,
(w) von Obesitas
(x) von kardiovasculären Erkrankungen wie Atherosclerose, Herzinsuffizienz, Herzklappenerkrankungen, zur Regulation des Blutdrucks bei Hyper- oder Hypotonie, oder zur Behandlung von pulmonaler arterieller Hypertension,
(y) von neuropsychatrischen Erkrankungen wie Drogenmissbrauch, Depression, Schizophrenie oder Angstzuständen und
(z) von Schlafstörungen wie z. B. Schlaflosigkeit.

Neben der Eignung als Humantherapeutika, sind diese Substanzen auch nützlich in der veterinärmedizinischen Therapie von Haustieren, exotischen Tieren und Nutztieren.

### KOMBINATIONEN

Zur Behandlung von Schmerzen kann es vorteilhaft sein, die erfindungsgemässen Verbindungen mit belebenden Stoffen wie Koffein oder anderen schmerzlindernden Wirkstoffen zu kombinieren. Stehen zur Behandlung der Ursache der Schmerzen geeignete Wirkstoffe zur Verfügung, so können diese mit den erfindungsgemässen Verbindungen kombiniert werden. Sind unabhängig von der Schmerzbehandlung auch noch weitere medizinische Behandlungen angezeigt, zum Beispiel gegen Bluthochdruck oder Diabetes, so können auch die dafür nötigen Wirkstoffe mit den erfindungsgemässen Verbindungen kombiniert werden.

Für eine Kombinationstherapie kommen beispielsweise die folgenden Verbindungen in Frage:
1. **Nicht-steroidale Antirheumatika (NSAR)**: einschließlich COX - Inhibitoren wie Propionsäure-Derivate (Alminoprofen, Benoxaprofen, Bucloxinsäure, Carprofen, Fenhufen, Fenoprofen, Fiuprofen, Fiulbiprofen, Ibuprofen, Indoprofen, Ketoprofen, Miroprofen, Naproxen ,Oxaprozin, Pirprofen, Pranoprofen, Suprofen, Tiaprofensäure, Tioxaprofen), Essigsäure-Derivate (Indomethacin, Acemetacin, Alclofenac, Clidanac, Diclofenac, Fenflofenac, Fentiazac, Furofenac, Ibufenac, Isoxepac, Oxpinax, Sulindac, Tiopinac, Tolmetin, Zidometacin, Zomepirac), Fenaminsäure-Derivate (Meclofenaminsäure, Mefenaminsäure, Tolfenaminsäure), Biphenyl-Carboxylsäure-Derivate, Oxicame (Isoxicam, Meloxicam, Piroxicam, Sudoxicam und Tenoxicam), Salicylsäure-Derivate (Acetylsalicylsäure, Sulfasalazin, Mesalazin, Olsalazin), und Pyrazolone (Apazon, Bezpiperylon, Feprazon, Mofebutazon, Oxyphenbutazon, Phenylbutazon, Propyphenazon und Metamizol), und Coxibe (Celecoxib, Valdecoxib, Rofecoxib, Etoricoxib),
2. **Opiat Rezeptor Agonisten** wie z. B. Morphin, Propoxyphen (Darvon), Tramadol, Buprenorphin,
3. **Cannabinoid Agonisten** wie z.B. GW-1000, KDS-2000, SAB-378, SP-104, NVP001, GW-843166, GW-842166X, PRS-211375,
4. **Natriumkanalblocker** wie z.B. Carbamazepin, Mexiletin, Lamotrigin, Pregabalin, Tectin, NW-1029, CGX-1002,
5. **N-Typ Calciumkanalblocker** wie z.B. Ziconitid, NMED-160, SP1-860,
6. **Serotonerge und noradrenerge Modulatoren** wie z.B. SR-57746, Paroxetin, Duloxetin, Clonidin, Amitriptylin, Citalopram, Flibanserin,
7. **Corticosteroide** wie z.B. Betamethason, Budesonid, Cortison, Dexamethason, Hydrocortison, Methylprednisolon, Prednisolon, Prednison und Triamcinolon.
8. **Histamin H1-Rezeptorantagonisten** wie z.B. Bromophthiramint, Chlorpheniramin, Dexchlorpheniramin, Triprolidin, Clemastin, Diphenhydramin, Diphenylpyralin, Tripelennamin, Hydroxyzin, Promethazin, Trimeprazin, Azatadin, Cyproheptadin, Antazolin, Pheniramin, Pyrilamin, Astemizol, Terfenadin, Loratadin, Cetirizin, Desloratadin, fexofenadin, Levocetirizin,
9. **Histamin H2-Rezeptor Antagonisten** wie z.B. Cimetidin, Famotidin, und Ranitidin,
10. **Protonenpumpenhemmer** wie z.B. Omeprazol, Pantoprazol, Esomeprazol,
11. **Leukotrien Antagonisten und 5-Lipoxygenasehemmer** wie z.B. Zafirlukast, Montelukast, Pranlukast und Zileuton,
12. **Lokalanästhetika** wie z.B. Lidocain, Ambroxol,
13. **VR1 Agonisten und Antagonisten** wie z.B. NGX-4010, WL-1002, ALGRX-4975, WL-10001, AMG-517,
14. **Nikotinrezeptor Agonisten** wie z.B. ABT-202, A-366833, ABT-594, BTG-102, A-85380, CGX1204,
15. **P2X3-Rezeptor Antagonisten** wie z.B. A-317491, ISIS-13920, AZD-9056,
16. **NGF Agonisten und Antagonisten** wie z.B. RI-724, RI-1024, AMG-819, AMG-403, PPH 207,
17. **NK1 und NK2 Antagonisten** wie z.B. DA-5018, R-116301, CP-728663, ZD-2249,
18. **NMDA Antagonisten** wie z.B. NER-MD-11, CNS-5161, EAA-090, AZ-756, CNP-3381,
19. **Kaliumkanal Modulatoren** wie z.B. CL-888, ICA-69673, Retigabin,
20. **GABA Modulatoren** wie z.B. Lacosamid,
21. **Anti-Migräne Therapeutika** wie z.B. Sumatriptan, Zolmitriptan, Naratriptan, Eletriptan, Telcagepant;
22. **iNOS-Inhibitoren** wie z.B. GSK 274150,
23. **CCR2-Antagonisten** wie z.B. PF-4136309, BMS-741672,
24. **Anticonvulsiva** wie z.B. Pregabalin oder Gabapentin.

Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils 1 bis 3 x täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel ¹H-NMR- und Massenspektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser. Soweit nicht anders vermerkt sind die bei den Aufarbeitungen der Reaktionslösungen verwendeten Säure-, Basen- und Salzlösungen wässrige Systeme der angegebenen Konzentrationen.
Zu chromatographischen Reinigungen wurde Kieselgel der Firma Millipore (MATREX™, 35 bis 70 µm) oder Alox (E. Merck, Darmstadt, Aluminiumoxid 90 standardisiert, 63 bis 200 µm, Artikel-Nr: 1.01097.9050) verwendet.

In den Versuchsbeschreibungen werden die folgenden Abkürzungen verwendet:
- CDI: 1,1'-Carbonyldiimidazol
- DC: Dünnschichtchromatogramm
- DIPEA: Diisopropylethylamin
- DMAP: 4-Dimethylaminopyridin
- DMF: Dimethylformamid
- DMSO: Dimethylsulfoxid
- HATU: O-(7-Azabenzotriazol-1-yl)-*N,N,N',N'*-tetramethyluroniumhexafluorphosphat
- *tert*: tertiär
- TBTU: 2-(1*H*-Benzotriazol-1-yl)-1,1,3,3-tetramethyluronium-tetrafluorborat
- THF: Tetrahydrofuran

Folgende analytische HPLC-Methoden wurden verwendet:

| | | | | |
|---|---|---|---|---|
| Methode 1: | Säule: | Zorbax Stable Bond C18, 3.5 µM, 4.6 x 75 mm | | |
| | Detektion: | 230 - 360 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 95.0 | 5.0 | 1.6 |
| | 0.1 | 95.0 | 5.0 | 1.6 |
| | 4.5 | 10.0 | 90.0 | 1.6 |
| | 5.09 | 10.0 | 90.0 | 1.6 |
| | 5.5 | 90.0 | 10.0 | 1.6 |

| | | | | |
|---|---|---|---|---|
| Methode 2: | Säule: | Interchim Strategy C18, 5 µM, 4.6 x 50 mm | | |
| | Detektion: | 220 - 320 nm | | |
| | Fließmittel A: | Wasser / 0.1 % TFA | | |
| | Fließmittel B: | Acetonitril | | |
| | Gradient: | | | |

| | Zeit in min | %A | %B | Flussrate in mL/min |
|---|---|---|---|---|
| | 0.0 | 95.0 | 5.0 | 3.0 |
| | 0.3 | 95.0 | 5.0 | 3.0 |
| | 2.0 | 2.0 | 98.0 | 3.0 |
| | 2.4 | 2.0 | 98.0 | 3.0 |
| | 2.45 | 95.0 | 5.0 | 3.0 |
| | 2.8 | 95.0 | 5.0 | 3.0 |

| | | | | |
|---|---|---|---|---|
| Methode 3: | Säule: | Merck Chromolith^{™} Flash RP18e, 4.6 x 25 mm | | |
| | Detektion: | 210 - 400 nm | | |
| | Fließmittel A: | Wasser / 0.1 % TFA | | |
| | Fließmittel B: | Acetonitril / 0.1 % TFA | | |
| | Gradient: | | | |

| | Zeit in min | %A | %B | Flussrate in mL/min |
|---|---|---|---|---|
| | 0.0 | 95.0 | 5.0 | 2.5 |
| | 0.2 | 95.0 | 5.0 | 2.5 |
| | 1.5 | 2.0 | 98.0 | 2.5 |
| | 1.7 | 2.0 | 98.0 | 2.5 |
| | 1.9 | 95.0 | 5.0 | 2.5 |
| | 2.2 | 95.0 | 5.0 | 2.5 |

| | | | | |
|---|---|---|---|---|
| Methode 4: | Säule: | YMC-Pack ODS-AQ, 3.0 µM, 4.6 x 75 mm | | |
| | Detektion: | 230 - 360 nm | | |
| | Fließmittel A: | Wasser / 0.1 % Ameisensäure | | |
| | Fließmittel B: | Acetonitril / 0.1 % Ameisensäure | | |
| | Gradient: | | | |

| | Zeit in min | %A | %B | Flussrate in mL/min |
|---|---|---|---|---|
| | 0.0 | 95.0 | 5.0 | 1.6 |
| | 4.5 | 10.0 | 90.0 | 1.6 |
| | 5.0 | 10.0 | 90.0 | 1.6 |
| | 5.5 | 90.0 | 10.0 | 1.6 |

### Herstellung der Endverbindungen

### Beispiel 1

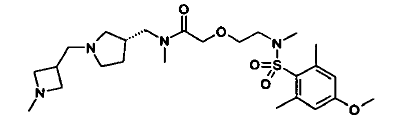

{2-[(4-Methoxy-2,6-dimethyl-benzolsulfonyl)-methyl-amino]-ethoxy}-essigsäure (150 mg, 0.45 mmol), TBTU (151 mg, 0.45 mMol) und Diisopropylethylamin (146 mg, 1.13 mMol) wurden in 3ml DMF gelöst und 10 Minuten bei Raumtemperatur gerührt. Dann wurde [1-(1-Methyl-azetidin-3-ylmethyl)-pyrrolidin-3-ylmethyl]-methylamin (89 mg, 0.45 mMol) zugegeben und über Nacht weitergerührt. Das Lösungsmittel wurde danach abgedampft und das so erhaltene Rohprodukt chromatographisch gereinigt.
Ausbeute: 22% der Theorie
C₂₅H₄₂N₄O₅S x CF₃COOH (624.7)
[M+H]+ = 511
HPLC (Methode 3): Retentionszeit = 1.28 min

### Beispiel 2

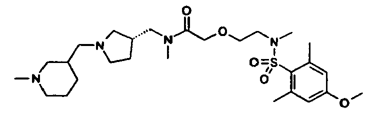

Hergestellt analog Beispiel 1 aus {2-[(4-Methoxy-2,6-dimethyl-benzolsulfonyl)-methyl-amino]-ethoxy}-essigsäure und [1-(1-Methyl-piperidin-3-ylmethyl)-pyrrolidin-3-ylmethyl]-methylamin.
Ausbeute: 16% der Theorie
C₂₇H₄₆N₄O₅S (538.7)
[M+H]+ = 539
HPLC (Methode 3): Retentionszeit = 1.30 min

### Beispiel 3

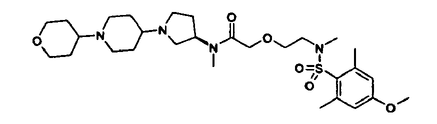

Hergestellt analog Beispiel 1 aus {2-[(4-Methoxy-2,6-dimethyl-benzolsulfonyl)-methyl-amino]-ethoxy}-essigsäure und {1-[1-(Tetrahydropyran-4-yl)-pipieridin-4-yl]-pyrrolidin-3-yl}-methylamin.
Ausbeute: 42% der Theorie
C₂₉H₄₈N₄O₆S x 2HCl (653.7)
[M+H]+ = 581
HPLC (Methode 4): Retentionszeit = 2.36 min

### Beispiel 4

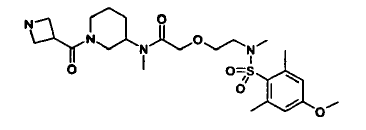

4a) 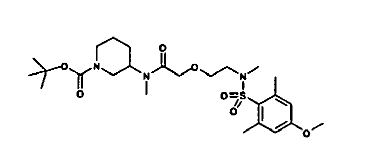 Hergestellt analog Beispiel 1 aus {2-[(4-Methoxy-2,6-dimethyl-benzolsulfonyl)-methyl-amino]-ethoxy}-essigsäure und 3-Methylamino-piperidin-1-carbonsäure-tert-butylester.
   Ausbeute: 98% der Theorie
   C₂₅H₄₁N₃O₇S (527.7)
   [M+H]+ = 528
   HPLC (Methode 4): Retentionszeit = 2.47 min
4b) 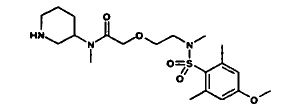 3-[(2-{2-[(4-Methoxy-2,6-dimethyl-benzolsulfonyl)-methyl-amino]-ethoxy}-acetyl)-methyl-amino]-piperidin-1-carbonsäure-tert-butylester (Produkt aus Beispiel 4a, 1.1 g, 1.97 mMol) wurde in 10 mL Dichlormethan gelöst, mit 6.5 mL Trifluoressigsäure versetzt und eine Stunde bei Raumtemperatur gerührt. Dann wurde das Gemisch im Vakuum bis zur Trockne eingedampft, der Rückstand in 50 mL Essigsäureethylester gelöst, dreimal mit je 25 mL 1 M Natronlauge und einmal mit 25 mL gesättigter Natriumchloridlösung extrahiert. Die vereinigten wässrigen Extrakte wurden dreimal mit je 30 mL Essigsäureethylester extrahiert und die vereinigten organischen Extrakte anschließend über Magnesiumsulfat getrocknet und eingeengt.
   Ausbeute: 99% der Theorie
   C₂₀H₃₃N₃O₅S (427.6)
   [M+H]+ = 528
   HPLC (Methode 3): Retentionszeit = 1.49 min
4c) 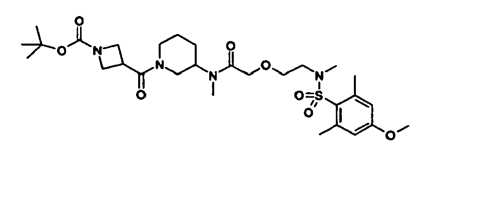 Hergestellt analog Beispiel 1 aus 2-{2-[(4-Methoxy-2,6-dimethyl-benzolsulfonyl)-methyl-amino]-ethoxy}-*N*-methyl-*N*-piperidin-3-yl-acetamid (Produkt aus Beispiel 4b) und 1-tert-Butyloxycarbonyl-azetidin-3-carbonsäure.
   Ausbeute: 96% der Theorie
   C₂₉H₄₆N₄O₈S (610.8)
   HPLC (Methode 3): Retentionszeit = 2.28 min.
4d) 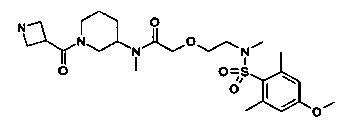 3-{3-[(2-{2-[(4-Methoxy-2,6-dimethyl-benzolsulfonyl)-methyl-amino]-ethoxy}-acetyl)-methyl-amino]-piperidin-1-carbonyl}-azetidin-1-carbonsäure-tert-butylester (Produkt aus Beispiel 4c, 217 mg, 0.36 mmol) wurden in 3 mL Dichlormethan gelöst, mit 2.5 mL Trifluoressigsäure versetzt und drei Stunden bei Raumtemperatur gerührt. Dann wurde das Reaktionsgemisch bis zur Trockne eingedampft und das so erhaltene Rohprodukt chromatographisch gereinigt.
   Ausbeute: 85% der Theorie
   C₂₄H₃₈N₄O₆S x CF₃COOH (624.7)
   [M+H]+ = 511
   HPLC (Methode 3): Retentionszeit = 1.55 min

### Beispiel 5

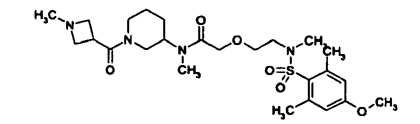

*N*-[1-(Azetidin-3-carbonyl)-piperidin-3-yl]-2-{2-[(4-methoxy-2,6-dimethylbenzolsulfonyl)-methyl-amino]-ethoxy}-*N*-methyl-acetamid (Produkt aus Beispiel 4, 293 mg, 0.57 mMol) wurde in 3 mL Dioxan gelöst, dann 0.13 mL einer 37%igen wässrigen FormaldehydLösung hinzugegeben und 30 Minuten bei Raumtemperatur gerührt. Anschließend wurden 304 mg (1.44 mMol) Natriumtriacetoxyborhydrid hinzugefügt und weitere 5 Stunden bei Raumtemperatur gerührt. Dann wurde das Reaktionsgemisch bis zur Trockne eingedampft und das so erhaltene Rohprodukt chromatographsch gereingt.
Ausbeute: 6.3% der Theorie
C₂₅H₄₀N₄O₆S (524.7)
[M+H]+ = 525
HPLC (Methode 3): Retentionszeit = 1.56 min

### Beispiel 6

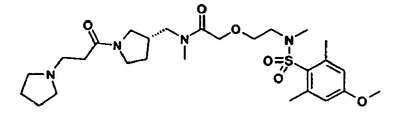

Hergestellt analog Beispiel 1 aus {2-[(4-Methoxy-2,6-dimethyl-benzolsulfonyl)-methyl-amino]-ethoxy}-essigsäure und 1-(3-Methylaminomethyl-pyrrolidin-1-yl)-3-pyrrolidin-1-yl-propan-1-on.
Ausbeute: 21% der Theorie
C₂₇H₄₄N₄O₆S (552.7)
[M+H]+ = 553
HPLC (Methode 3): Retentionszeit = 1.62 min

### Beispiel 7

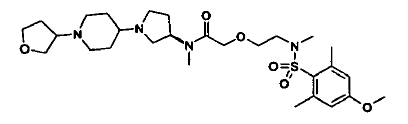

Hergestellt analog Beispiel 1 aus {2-[(4-Methoxy-2,6-dimethyl-benzolsulfonyl)-methyl-amino]-ethoxy}-essigsäure und Methyl-{1-[1-(tetrahydrofuran-3-yl)-piperidin-4-yl]-pyrrolidin-3-yl}-amin.
Ausbeute: 42% der Theorie
C₂₈H₄₆N₄O₆S x 2HCl (639.7)
[M+H]+ = 567
HPLC (Methode 4): Retentionszeit = 2.99 min

### Beispiel 8

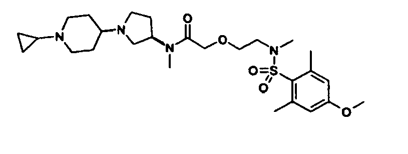

Hergestellt analog Beispiel 1 aus {2-[(4-Methoxy-2,6-dimethyl-benzolsulfonyl)-methyl-amino]-ethoxy}-essigsäure und [1-(1-Cyclopropyl-piperidin-4-yl)-pyrrolidin-3-yl]-methylamin.
Ausbeute: 46% der Theorie
C₂₇H₄₄N₄O₅S x 2HCl (609.7)
[M+H]+ = 537
HPLC (Methode 1): Retentionszeit = 3.10 min

### Beispiel 9

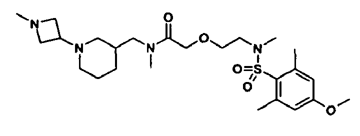

Hergestellt analog Beispiel 1 aus {2-[(4-Methoxy-2,6-dimethyl-benzolsulfonyl)-methyl-amino]-ethoxy}-essigsäure und Methyl-[1-(1-methyl-azetidin-3-yl)-piperidin-3-yl-methyl]-amin.
Ausbeute: 76% der Theorie
C₂₅H₄₂N₄O₅S x CF₃COOH (624.7)
[M+H]+ = 511
HPLC (Methode 3): Retentionszeit = 1.34 min

### Beispiel 10

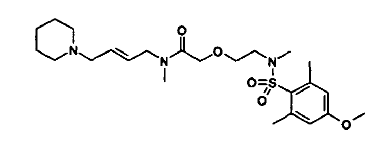

Hergestellt analog Beispiel 1 aus {2-[(4-Methoxy-2,6-dimethyl-benzolsulfonyl)-methyl-amino]-ethoxy}-essigsäure und Methyl-(4-piperidin-1-yl-but-2-enyl)-amin.
Ausbeute: 92% der Theorie
C₂₄H₃₉N₃O₅S x CF₃COOH (595.7)
[M+H]+ = 482
HPLC (Methode 2): Retentionszeit = 1.37 min

### Beispiel 11

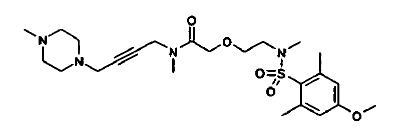

Hergestellt analog Beispiel 1 aus {2-[(4-Methoxy-2,6-dimethyl-benzolsulfonyl)-methyl-amino]-ethoxy}-essigsäure und Methyl-[4-(4-methyl-piperazin-1-yl)-but-2-inyl]-amin.
Ausbeute: 43% der Theorie
C₂₄H₃₈N₄O₅S x CF₃COOH (608.7)
[M+H]+ = 495
HPLC (Methode 2): Retentionszeit = 1.27 min

### Beispiel 12

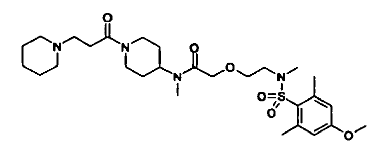

Hergestellt analog Beispiel 1 aus 2-[(4-Methoxy-2,6-dimethyl-benzolsulfonyl)-methyl-amino]-ethoxy}-essigsäure und 1-(4-Methylamino-piperidin-1-yl)-3-piperidin-1-yl-propan-1-on.
Ausbeute: 77% der Theorie
C₂₈H₄₆N₄O₆S x CF₃COOH (680.8)
[M+H]+ = 567
HPLC (Methode 3): Retentionszeit = 1.61 min

### Beispiel 13

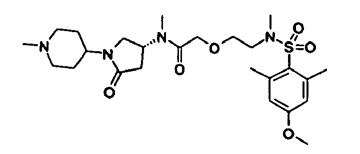

Hergestellt analog Beispiel 1 aus 2-[(4-Methoxy-2,6-dimethyl-benzolsulfonyl)-methyl-amino]-ethoxy}-essigsäure und 4-Methylamino-1-(1-methyl-piperidin-4-yl)-pyrrolidin-2-on.
Ausbeute: 19% der Theorie
C₂₅H₄₀N₄O₆S x CF₃COOH (638.7)
[M+H]+ = 525
HPLC (Methode 3): Retentionszeit = 1.66 min

Die nachfolgenden Beispiele beschreiben pharmazeutischer Darreichungsformen, die als Wirkstoff eine beliebige Verbindung der allgemeinen Formel I enthalten:

### Beispiel I

Trockenampulle mit 75 mg Wirkstoff pro 10 ml

### Zusammensetzung:

| | |
|---|---|
| Wirkstoff | 75.0 mg |
| Mannitol | 500 mg |
| Wasser für Injektionszwecke ad | 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 9 mm.

### Beispiel III

Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 12 mm.

### Beispiel IV

Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

### Beispiel V

Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Gr6Be 0 abgefüllt.

### Beispiel VI

Suppositorien mit 100 mg Wirkstoff

1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

## Patentansprüche

1. Verbindungen der allgemeinen Formel **I** 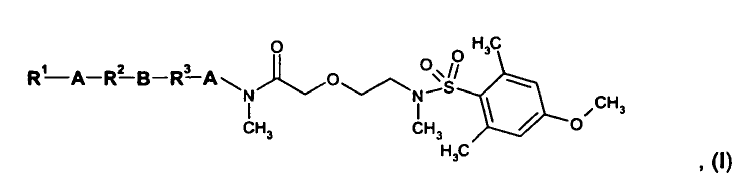 in der
**A**
(a) eine Bindung oder
(c) eine C₁₋₃-Alkylengruppe,
**B**
(a) eine Bindung,
(d) eine C₁₋₃-Alkylen- oder
(e) eine -C(O)-Gruppe,
**R¹**
(a) H,
(f) C₁₋₃-Alkyl,
(g) C₃₋₆-Cycloalkyl,
(h) einen 5- oder 6-gliedrigen, gesättigten Aza-Heterocyclus oder
(i) einen 5- oder 6-gliedrigen, gesättigten Oxa-Heterocyclus,
**R**²
(a) C₁₋₃-Alkylen,
(d) einen 4-bis 6-gliedrigen, gesättigten Aza-Heterocyclus, oder
(e) einen 6- bis 7-gliedrigen, gesättigten Diaza-Heterocyclus,
**R³**
(a) C₂₋₄-Alkenylen,
(d) C₂₋₄-Alkinylen oder
(e) einen 5- bis 6-gliedrigen, gesättigten Aza-Heterocyclus, in dem zusätzlich eine Methylengruppe durch eine Carbonylgruppe substituiert sein kann,
deren Enantiomere, deren Diastereomere, deren Gemische und deren Salze, insbesondere deren physiologisch verträgliche Salze mit organischen oder anorganischen Säuren oder Basen.

2. Verbindungen der allgemeinen Formel I gemäß Anspruch 1, in denen
**A** eine Bindung oder eine -CH₂-Gruppe,
**B** eine Bindung, eine -CH₂- oder -C(O)-Gruppe,
**R¹** H, H₃C- oder eine Gruppe ausgewählt aus 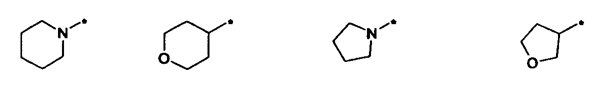 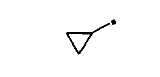
**R²** eine Gruppe ausgewählt aus 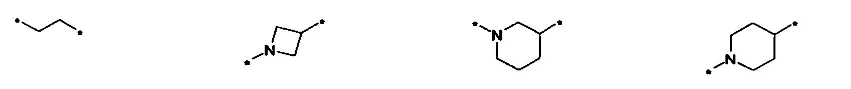 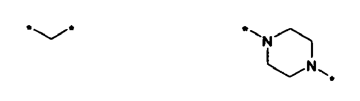 und
**R³** eine Gruppe ausgewählt aus 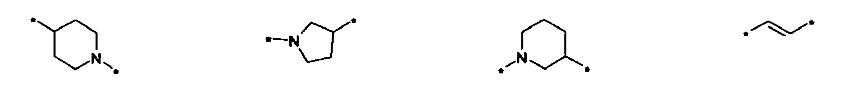 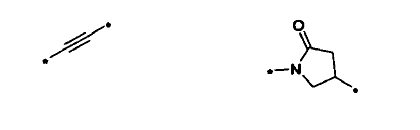
bedeutet,
deren Enantiomere, deren Diastereomere, deren Gemische und deren Salze, insbesondere deren physiologisch verträgliche Salze mit organischen oder anorganischen Säuren oder Basen.

3. Folgende Verbindungen der allgemeinen Formel I gemäß Anspruch 1:
| **Beispiel** | **Struktur** |
|---|---|
| (1) | 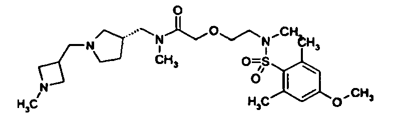 |
| (2) | 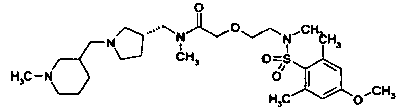 |
| (3) | 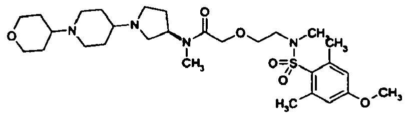 |
| (4) | 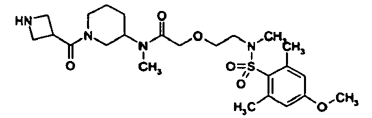 |
| (5) | 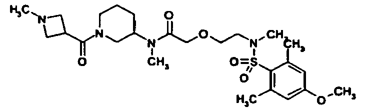 |
| (6) | 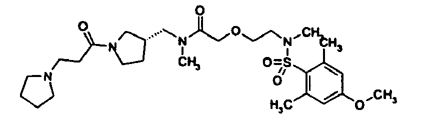 |
| (7) | 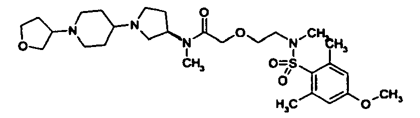 |
| (8) | 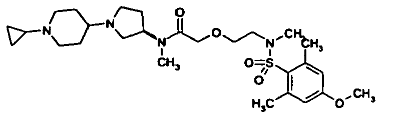 |
| (9) | 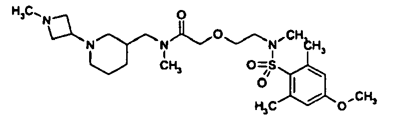 |
| (10) | 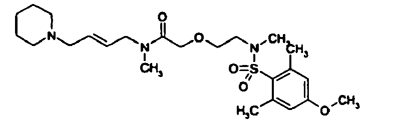 |
| (11) | 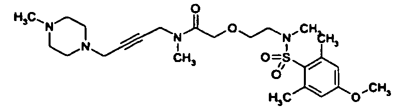 |
| (12) | 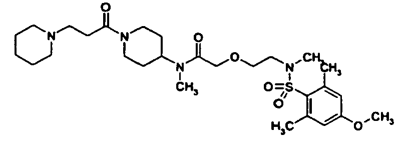 |
| (13) | 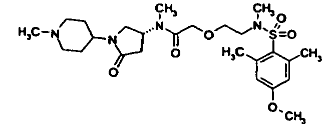 |
deren Enantiomere, deren Diastereomere, deren Gemische und deren Salze, insbesondere deren physiologisch verträgliche Salze mit organischen oder anorganischen Säuren oder Basen.

4. Physiologisch verträgliche Salze der Verbindungen nach einem der Ansprüche 1 bis 3 mit anorganischen oder organischen Säuren oder Basen.

5. Arzneimittel, enthaltend eine Verbindung nach mindestens einem der Ansprüche 1 bis 3 oder ein physiologisch verträgliches Salz gemäß Anspruch 4 neben gegebenenfalls einem oder mehreren inerten Trägerstoffen und/oder Verdünnungsmitteln.

6. Verwendung einer Verbindung nach mindestens einem der Ansprüche 1 bis 4 zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen.

7. Verfahren zur Herstellung eines Arzneimittels gemäß Anspruch 5, **dadurch gekennzeichnet, dass** auf nichtchemischem Weg eine Verbindung nach mindestens einem der Ansprüche 1 bis 4 in einen oder mehrere inerte Trägerstoffe und/oder Verdünnungsmittel eingearbeitet wird.

## Claims

1. Compounds of general formula **I** 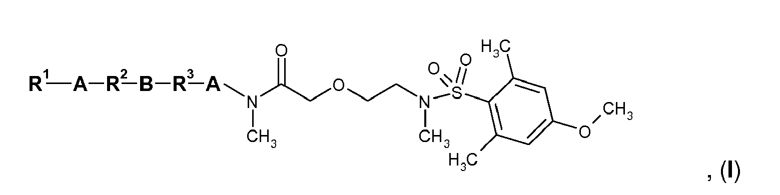 wherein
**A** denotes
(a) a bond or
(c) a C₁₋₃-alkylene group,
**B** denotes
(a) a bond,
(d) a C₁₋₃-alkylene or
(e) a -C(O)- group,
**R**¹ denotes
(a) H,
(f) C₁₋₃-alkyl,
(g) C₃₋₆-cycloalkyl,
(h) a 5- or 6-membered, saturated aza-heterocycle or
(i) a 5- or 6-membered, saturated oxa-heterocycle,
**R**² denotes
(a) C₁₋₃-alkylene,
(d) a 4-to 6-membered, saturated aza-heterocycle, or
(e) a 6- to 7-membered, saturated diaza-heterocycle,
**R**³ denotes
(a) C₂₋₄-alkenylene,
(d) C₂₋₄-alkynylene or
(e) a 5- to 6-membered, saturated aza-heterocycle, wherein additionally a methylene group may be substituted by a carbonyl group,
the enantiomers, the diastereomers, the mixtures and the salts thereof, particularly the physiologically acceptable salts thereof with organic or inorganic acids or bases.

2. Compounds of general formula **I** according to claim 1, wherein
**A** denotes a bond or a -CH₂- group,
**B** denotes a bond, a -CH₂- or -C(O)- group,
**R**¹ denotes H, H₃C or a group selected from 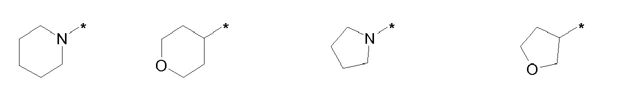 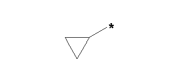
**R**² denotes a group selected from 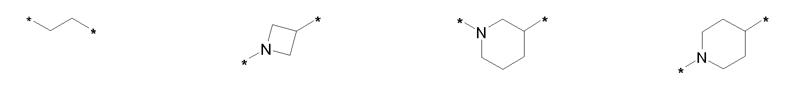 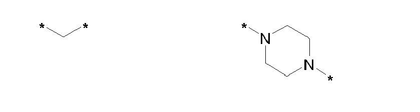 and
**R**³ denotes a group selected from 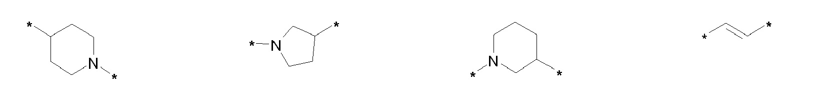 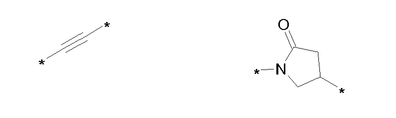
the enantiomers, the diastereomers, the mixtures and the salts thereof, particularly the physiologically acceptable salts thereof with organic or inorganic acids or bases.

3. The following compounds of general formula **I** according to claim 1:
| **Example** | **Structure** |
|---|---|
| (1) | 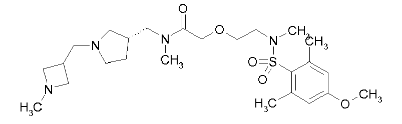 |
| (2) | 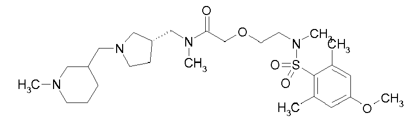 |
| (3) | 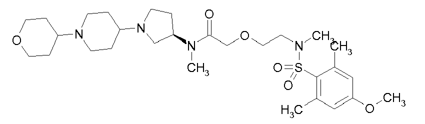 |
| (4) | 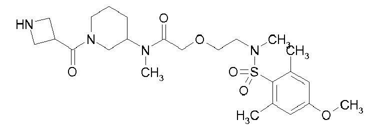 |
| (5) | 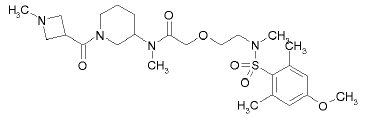 |
| (6) | 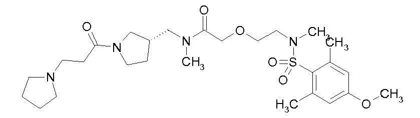 |
| (7) | 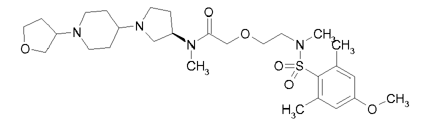 |
| (8) | 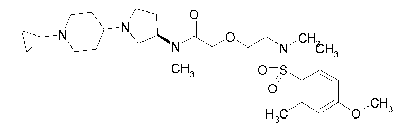 |
| (9) | 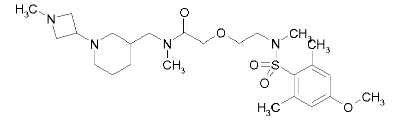 |
| (10) | 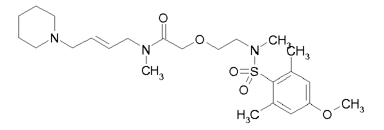 |
| (11) | 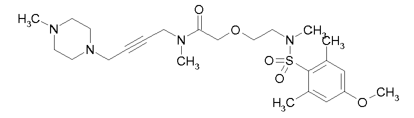 |
| (12) | 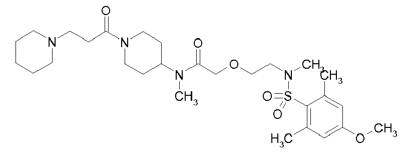 |
| (13) | 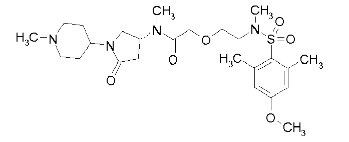 |
the enantiomers, the diastereomers, the mixtures and the salts thereof, particularly the physiologically acceptable salts thereof with organic or inorganic acids or bases.

4. Physiologically acceptable salts of the compounds according to one of claims 1 to 3 with inorganic or organic acids or bases.

5. Medicament, containing a compound according to at least one of claims 1 to 3 or a physiologically acceptable salt according to claim 4 optionally together with one or more inert carriers and/or diluents.

6. Use of a compound according to at least one of claims 1 to 4 for preparing a medicament for the acute and prophylactic treatment of acute pain, visceral pain, neuropathic pain, inflammatory/pain receptor-mediated pain, tumour pain and headache diseases.

7. Process for preparing a medicament according to claim 5, **characterised in that** by a non-chemical method a compound according to at least one of claims 1 to 4 is incorporated in one or more inert carriers and/or diluents.

## Revendications

1. Composés de formule générale I 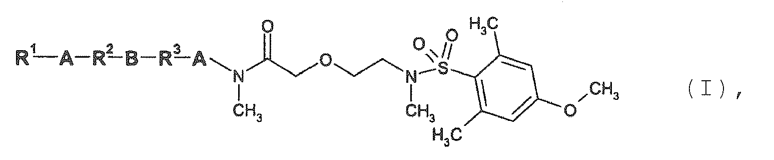 dans laquelle
**A** est
(a) une liaison ou
(c) un groupe alkylène en C₁₋₃,
**B** est
(a) une liaison,
(d) un groupe alkylène en C₁₋₃ ou
(e) un groupe -C(O),
**R**¹ est
(a) H,
(f) un groupe alkyle en C₁₋₃,
(g) un groupe cycloalkyle en C₃₋₆,
(h) un groupe azahétérocyclique saturé
de 5 ou 6 termes, ou
(i) un groupe oxahétérocyclique saturé de 5 ou 6 termes,
**R**² est
(a) un groupe alkylène en C₁₋₃,
(d) un groupe azahétérocyclique saturé de 4 à 6 termes, ou
(e) un groupe diazahétérocyclique saturé de 6 à 7 termes,
**R**³ est
(a) un groupe alcénylène en C₂₋₄,
(d) un groupe alcynylène en C₂₋₄ ou
(e) un groupe azahétérocyclique saturé de 5 à 6 termes dans lequel un groupe méthylène peut en plus être substitué par un groupe carbonyle,
leurs énantiomères, leurs diastéréoisomères, leurs mélanges et leurs sels, en particulier leurs sels physiologiquement compatibles avec des acides ou des bases organiques ou inorganiques.

2. Composés de formule générale I selon la revendication 1, dans lesquels
**A** est une liaison ou un groupe -CH₂,
**B** est une liaison ou un groupe -CH₂- ou -C(O),
**R**¹ est H, un groupe H₃C ou un groupe choisi parmi 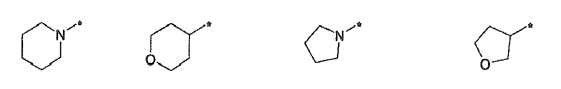 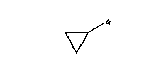
**R**² est un groupe choisi parmi 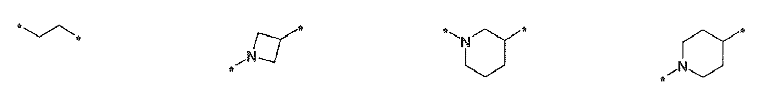 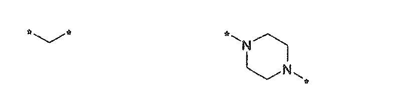 et
**R**³ est un groupe choisi parmi 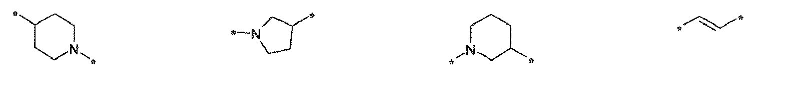 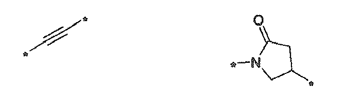
leurs énantiomères, leurs diastéréoisomères, leurs mélanges et leurs sels, en particulier leurs sels physiologiquement compatibles avec des acides ou des bases organiques ou inorganiques.

3. Composés suivants de formule générale I selon la revendication 1 :
| Exemple | Structure |
|---|---|
| (1) | 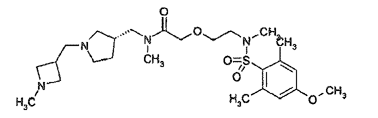 |
| (2) | 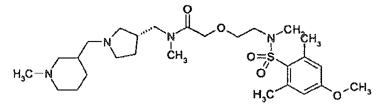 |
| (3) | 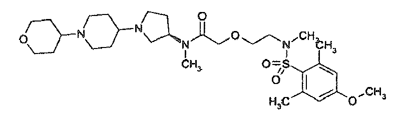 |
| (4) | 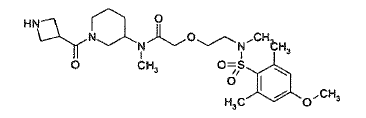 |
| (5) | 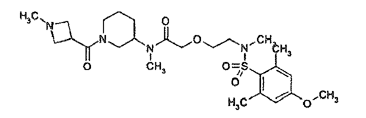 |
| (6) | 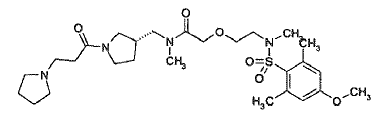 |
| (7) | 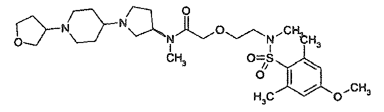 |
| (8) | 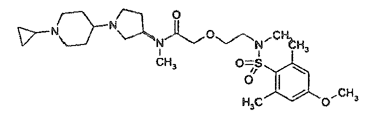 |
| (9) | 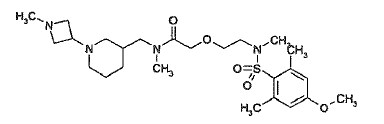 |
| (10) | 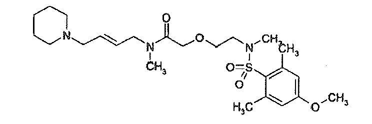 |
| (11) | 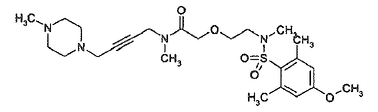 |
| (12) | 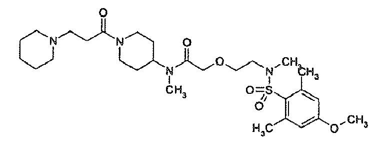 |
| (13) | 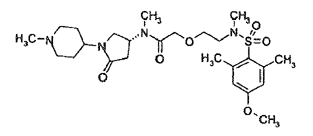 |
leurs énantiomères, leurs diastéréoisomères, leurs mélanges et leurs sels, en particulier leurs sels physiologiquement compatibles avec des acides ou des bases organiques ou inorganiques.

4. Sels physiologiquement compatibles des composés selon l'une des revendications 1 à 3 avec des acides ou des bases organiques ou inorganiques.

5. Médicament contenant un composé selon l'une au moins des revendications 1 à 3 ou un sel physiologiquement compatible selon la revendication 4, ainsi le cas échéant qu'un ou plusieurs véhicules et/ou diluants inertes.

6. Utilisation d'un composé selon l'une au moins des revendications 1 à 4 pour préparer un médicament destiné au traitement aigu et prophylactique
de douleurs aiguës, de douleurs intestinales, de douleurs neuropathiques, de douleurs inflammatoires/médiées par les récepteurs de la douleur, de douleurs tumorales et de pathologies migraineuses.

7. Procédé de préparation d'un médicament selon la revendication 5, **caractérisé en ce que** l'on incorpore par une voie non chimique un composé selon l'une au moins des revendications 1 à 4 dans un ou plusieurs véhicules et/ou diluants inertes.
